**Europäisches Patentamt**

**European Patent Office**

**Office européen des brevets**

(19)

(11) Veröffentlichungsnummer: **0 153 995**
**B1**

# EUROPÄISCHE PATENTSCHRIFT

(12)

(45) Veröffentlichungstag der Patentschrift:
31.05.89

(21) Anmeldenummer: **84112944.8**

(22) Anmeldetag: **07.10.80**

(51) Int. Cl.⁴: **A 61 L 2/20,** A 01 N 37/16,
**A 61 K 35/12**

(54) **Verfahren zur schonenden Sterilisation von biologischen Wirkstoffen, insbesondere von Organgeweben für therapeutische Zwecke gegenüber Mikroorganismen und Viren.**

(30) Priorität: **02.11.79 DE 2944278**

(43) Veröffentlichungstag der Anmeldung:
**11.09.85 Patentblatt 85/37**

(45) Bekanntmachung des Hinweises auf die Patenterteilung:
**31.05.89 Patentblatt 89/22**

(84) Benannte Vertragsstaaten:
**AT BE CH FR GB IT LI LU NL SE**

(56) Entgegenhaltungen:
**DE-B-2 004 553**
**FR-A-2 321 302**

**DIE PHARMAZIE, Nr. 31, Heft 7, 1976, Seiten 491-492,
VEB Verlag Volk und Gesundheit, Berlin, DE; M.
SPRÖSSIG et al.: "Untersuchungen über die
sterilisierende Wirkung von gasförmiger
Peressigsäure an papierverpacktem Material"
DIE PHARMAZIE, Nr. 29, Heft 2, 1974, Seiten 132-137,
VEB Verlag, Berlin, DE; M. SPRÖSSIG et al.:
"Versuche und Betrachtungen zur Sterilisation
thermolabiler Gegenstände mit Peressigsäure"
H.P. WEUFFEN: "Handbuch der Desinfektion und
Sterilisation", Band 1, 1972, Seiten 173-175, VEB
Verlag Volk und Gesundheit, Berlin, DE**

**Die Akte enthält technische Angaben, die nach
dem Eingang der Anmeldung eingereicht wurden**

(73) Patentinhaber: **Theurer, Karl, Prof. Dr.,
Brunnwiesenstrasse 21, D-7302 Ostfildern 1 (DE)**

(72) Erfinder: **Theurer, Karl, Prof. Dr.,
Brunnwiesenstrasse 21, D-7302 Ostfildern 1 (DE)**

(74) Vertreter: **Land, Addick Adrianus Gosling,
OCTROOIBUREAU ARNOLD & SIEDSMA P.O.
Box 18558, NL- 2502 EN The Hague (NL)**

(56) Entgegenhaltungen: (Fortsetzung)
**und die nicht in dieser Patentschrift enthalten sind.**

EP 0 153 995 B1

## Beschreibung

Die Erfindung betrifft ein Verfahren zur schonenden Sterilisation von biologischen Wirkstoffen, insbesondere von Organgeweben für therapeutische Zwecke gegenüber Mikroorganismen und Viren, die einem gesteuerten chemischen Aufschluß unter Verwendung von Dämpfen und chemischen Reagenzien unterworfen werden, bei welchen man die Pulverförmigen Substrate mit dem den Aufschluß bewirkenden Reagenz, dessen Siedepunkt bei atmosphärischem Druck über der Normaltemperatur liegt, insbesondere konz. Schwefelsäure, Essigsäure, Ameisensäure oder Diäthylamin in einem abgeschlossenen System zusammen, aber getrennt voneinander, einem solchen Vakuum aussetzt, welches die Überführung des aufschließenden Mittels in die dampfförmige Phase ermöglicht, und, nachdem der gewünschte Aufschluß des Substrates erreicht ist, das nicht verbrauchte Reagenz wieder entfernt, wobei man insbesondere von tiefgekühlten und pulverisierten organischen Geweben ausgeht, und man das dampfförmige chemische Reagenz aus einer vorbestimmten Menge durch Absenken des Vakuums zunächst auf dem Substrat niederschlägt, und nach der gewünschten Einwirkung das überschüssige Reagenz durch Erhöhung des Vakuums wieder entfernt, welches dadurch gekennzeichnet ist, daß man die Substrate im Vakuum Dämpfen von Persäuren oder von Mischungen von Persäuren und den entsprechenden konzentrierten Säuren aussetzt.

Eine besondere Ausgestattung des erfindungsgemäßen Verfahrens ist dadurch gekennzeichnet, daß die Säuredämpfe ein Mischungsverhältnis von 1 Teil Persäure zu 1 - 4 Teilen des entsprechenden Säureanhydrids aufweisen.

Die Gewinnung steriler Arzneimittel aus biologischen Geweben, insbesondere aus Organgeweben, stößt auf Schwierigkeiten, weil mit den üblichen Desinfektionsmitteln nicht ohne starke Denaturierung und therapeutische Wirkungseinbuße absolute Sterilität erreicht werden kann. Deshalb wurden von der Gesundheitsbehörde für die Herstellung solcher Arzneimittel besonders erschwerende Auflagen gemacht, wie z. B. Haltung isolierter Tierherden und Aufzucht unter Sterilbedingungen. Diese Auflagen führen zu einer kaum tragbaren Verteuerung der Produkte, ohne daß damit eine absolute Gewähr für Sterilität, insbesondere gegen Viren, erreicht werden könnte. Die Mitverwendung von Antibiotika und bzw. oder Chemotherapeutika, die imstande sind, Viren zu inaktivieren, ist hingegen mit dem Risiko der Allergenisierung des Patienten behaftet.

Es konnte nun in Versuchen mit Organpulvern aus virusinfizierten Hühnerembryonen nachgewiesen werden, daß mit einem modifizierten Verfahren zum gesteuerten chemischen Aufschluß, von Organen für therapeutische Zwecke, das im Patentanspruch definiert ist, Sterilität erreicht wird. Das Verfahren zum gesteuerten chemischen Aufschluß von organischen Stoffen und biologischen Geweben für therapeutische Zwecke ist Gegenstand der DE-PS-1 090 821 und betrifft, daß man die pulverförmigen Substrate mit dem den Aufschluß bewirkenden Reagenz, dessen Siedepunkt bei atmosphärischem Druck über der Normaltemperatur liegt, insbesondere konz. Schwefelsäure, Essigsäure, Ameisensäure oder Diäthylamin in einem abgeschlossenen System zusammen, oder getrennt voneinander, einem solchen Vakuum aussetzt, welches die Überführung des aufschließenden Mittels in die dampfförmige Phase ermöglicht, und, nachdem der gewünschte Aufschluß des Substrates erreicht ist, das nicht verbrauchte Reagenz wieder entfernt, wobei man insbesondere von tiefgekühlten und pulverisierten organischen Geweben ausgeht, und man das dampfförmige chemische Reagenz aus einer vorbestimmten Menge durch Absenken des Vakuums zunächst auf dem Substrat niederschlägt, und nach der gewünschten Einwirkung das überschüssige Reagenz durch Erhöhung des Vakuums wieder entfernt. Das Verfahren wurde vorzugsweise dadurch modifiziert, daß die Substrate bei einem Restwassergehalt von 5 bis 30 % gegenüber einem Normalwassergehalt von 60 bis 87 % von Frischgeweben, der Einwirkung von Säuredämpfen im Vakuum ausgesetzt werden, denen die entsprechenden Persäuren, besonders Ameisensäure, Essigsäure oder Schwefelsäure, in einem Mischungsverhältnis von 1 Teil Persäure zu 1 bis 4 Teilen des entsprechenden Säureanhydrids zugesetzt sind. Auch können Dämpfe von Säuren verwendet werden, denen bei der Herstellung eine geringere Menge von Wasserstoffperoxyd zugesetzt wird, als zur Herstellung der Persäure erforderlich wäre, z. B. zu 10 Teilen einer 98-%-igen Ameisensäure, 0,1 bis 0,8 % von 30-%-igen Wasserstoffperoxyd zusammen mit 0,5 % konz. Schwefelsäure, anstatt 10 Teilen konz. Ameisensäure mit 1 Teil Wasserstoffperoxyd, und zu 10 Teilen Essigsäureanhydrid nur 0,5 bis 4 Teile frisches 30-%-iges Wasserstoffperoxyd anstatt 5 Teile desselben. Der Vorteil dieses Verfahrens ist, daß die "partiellen" Persäuren weniger aggressiv und besser manipulierbar, d. h. weniger explosiv sind.

Bei der Bedampfung des Substrates soll die Menge der Säure, die auf dem Substrat kondensiert, der Einwirkung einer 0,5- bis 2-%-igen Persäure entsprechen. Der Kondensationsvorgang kann, wenn erforderlich mehrmals wiederholt werden, indem man das Vakuum kurz absenkt, bevor man erneut die Säuredämpfe einströmen und auf dem Substrat kondensieren läßt. Die Einwirkung kann bei nicht begrenzten Säuremengen einige Minuten, und bei Säuremengen, die auf die Substratmenge abgestimmt sind, bis zu mehreren Stunden betragen.

Der Nachweis der Sterilität des Substrats erfolgt durch die bekannten Methoden der Mikrobiologie und Virologie über die Züchtung der Viren in Hühnereiern oder in Zellkulturen. Der Nachweis der noch erhaltenen biologischen Wirkung der Präparate erfolgt durch Bioassay an Zellkulturen (V. Paffenholz und K. Theurer: Einfluß von makromolekularen Organsubstanzen auf menschliche Zellen in vitro, Der Kassenarzt H. 27, September 1978 und H. 19, Mai 1979, S. 876 - 877) sowie durch Tierversuche.

Das Verfahren führt zu keinen toxisch wirkenden Produkten, wie z. B. Heterozyklen, weil die erhaltenen Bruchstücke wegen des fehlenden Wassers nicht miteinander reaggregieren oder konjugieren. Die in geringem Ausmaße entstehenden Peroxyde bewirken in den Substraten bei der therapeutischen Anwendung eine erwünschte Reizwirkung, ähnlich wie bei der therapeutischen Anwendung von Ozon. Auch läßt sich bei diesem Verfahren die nicht zw Reaktion gekommene Säure durch Erhöhung des Vakuums absaugen. Sonst kann aber auch eine Neutralisierung durch Einblasen von Alkalidämpfen, z. B. von Merkaptoäthanol und erneutes Absaugen erfolgen. Durch die gleichzeitige Reduktionswirkung läßt sich die Oxydationswirkung zum Teil wieder rückgängig machen.

Die Anwendung von Dämpfen von Persäuren erfordert säureresistente Kunststoffschläuche und -dichtungen. Der Rezipient für die Persäure kann zweckmäßigerweise durch einen Dreiwegehahn mit der Vakuumpumpe und andererseits dem Rezipienten für das Substrat verbunden werden, so daß man nach Erzeugung des Vakuums im Substrat-Rezipienten die Vakuumpumpen absperren und die Säuredämpfe einströmen lassen kann.

Es ist auch möglich, gefrorenes, nicht getrocknetes Gewebepulver, zunächst bis zum Erreichen der gewünschten Restfeuchtigkeit durch Gefriertrocknung oder in Anwesenheit hygroskopischer Stoffe bzw. von konzentrierter Schwefelsäure vorzutrocknen, bevor man den Sterilisationsvorgang einleiten Trockengewebe mit einer zu geringen Restfeuchtigkeit können durch Einleiten von Wasserdampf oder Anfeuchten vorbehandelt werden.

Über die Peressigsäure als Möglichkeit der "Kaltsterilisation" bei der Aufbereitung thermosensibler Instrumente haben K. Bansemir, H. Bellinger, K. Disch und W. Kästner in "Hygiene und Medizin" 4 (1979) 311 - 316 publiziert. Dabei wurde der sterilisierende Effekt einer 1- bis 2-%-igen Lösung auf Papova-Viren, Entero-Viren, Poliomyelitis Typ 1, als auch Coxsackie-Viren Typ B III und Hepatitis-B-Viren, nachgewiesen. Auch wurde über toxikologische Untersuchungen berichtet. Die Anwendung von Säurelösungen ist jedoch nicht mit dem beanspruchten Verfahren identisch, weil hier die Anwendung bei Raumtemperatur in Form von Säuredämpfen erfolgt, und beim Trocknungsvorgang die nicht zur Reaktion gekommene Säure wieder durch Absaugen beseitigt wird. Desgleichen werden nach Weyls, "Handbuch der Hygiene, Band VIII: Die Desinfektion", Barth, Leipzig 1922, S. 983 - 987, Persäuren nur in flüssiger Phase eingesetzt.

**Beispiel 1**

Das Beispiel zeigt die Inaktivierung von New Castle Disease Virus (NDV) in Hühnerfoeten und Eihäuten. Diese werden nach der Entnahme aus infizierten bebrüteten Hühnereiern in flüssigem Stickstoff tiefgefroren und pulverisiert. Das tiefgefrorene Pulver wird in dünner Schicht auf einer Petrischale verteilt und in einem Exsikkator über eine Schale mit konzentrierter Schwefelsäure gebracht. Der Exsikkator steht über einem Dreiwegehahn mit der Hochvakuumpumpe und einem Druckgefäß, in dem sich 10 ml einer Mischung von 1 Teil Peressigsäure mit 2 Teilen Essigsäure befindet, in Verbindung. Es wird nun zunächst das Exsikkatorgefäß 2 Stunden lang evakuiert, bis das gefrorene Pulver zu etwa 1/2 bis 1/4 getrocknet ist. Dann wird die Vakuumpumpe durch das Ventil abgekoppelt und der Rezipient mit Persäuremischung zum Exsikkator geöffnet, so daß die Säure verdampft und in den Exsikkator gelangt. Es hängt vom Volumen des Exsikkators ab, wieviel Säure verdampft. Gegebenenfalls muß wiederholt das Vakuum im Exsikkatorgefäß erneuert werden, damit die gesamte Säure verdampft. Die Einwirkungszeit beträgt 30 Minuten. Danach wird bis zur Volltrockenheit das Pulver abgesaugt. Es kann aber auch nach der Säureeinwirkung der Rezipient für Säure durch einen solchen mit Merkaptoäthanol ersetzt werden und dieses in gleicher Weise, wie vorher die Säure, auf das Gewebepulver zur Einwirkung gebracht werden, bevor man den Trocknungsvorgang durch erneutes Absaugen beendet. Auch kann anstelle der Trocknung über der hygroskopischen konz. Schwefelsäure die Gefriertrocknung mittels einer Kühlfalle erfolgen. Das gewonnene Trockenpulver erweist sich dann bei der virologischen Überprüfung als steril.

**Beispiel 2**

Es soll virusinfiziertes Trockenpulver sterilisiert werden. Dazu bringt man das Trockenpulver in einer Schichtdicke von 0,5 bis 1 cm auf Petrischalen, die in einen Exsikkator gebracht werden. Dieser wird nun evakuiert und dann Wasserdampf eingeleitet, der sich auf dem Substrat kondensiert und dieses befeuchtet. Es wird nun erneut evakuiert und dann die Dämpfe einer Säuremischung aus 1 Teil Perameisensäure mit 2 Teilen 98-%-iger Ameisensäure eingeleitet Im weiteren verfährt man wie Beispiel 1.

## Patentansprüche

1. Verfahren zur schonenden Sterilisation von biologischen Wirkstoffen, insbesondere von Organgeweben für therapeutische Zwecke gegenüber Mikroorganismen und Viren, die einem gesteuerten chemischen Aufschluß unter Verwendung von Dämpfen und chemischen Reagenzien unterworfen werden, bei welchen man die pulverförmigen Substrate mit dem den Aufschluß bewirkenden Reagenz, dessen Siedepunkt bei atmosphärischem Druck über der Normaltemperatur liegt, insbesondere konz. Schwefelsäure, Essigsäure, Ameisensäure oder Diäthylamin in einem abgeschlossenen System zusammen, aber getrennt voneinander, einem solchen Vakuum aussetzt, welches die Überführung des aufschließenden Mittels in die dampfförmige Phase ermöglicht, und, nachdem der gewünschte Aufschluß des Substrates erreicht ist, das nicht verbrauchte Reagenz wieder entfernt, wobei man insbesondere von tiefgekühlten und pulverisierten organischen Geweben ausgeht, und man das dampfförmige chemische Reagenz aus einer vorbestimmten Menge durch Absenken des Vakuums zunächst auf dem Substrat niederschlägt, und nach der gewünschten Einwirkung das überschüssige Reagenz durch Erhöhung des Vakuums wieder entfernt, dadurch gekennzeichnet, daß man die Substrate im Vakuum Dämpfen von Persäuren oder von Mischungen von Persäuren und den entsprechenden konzentrierten Säuren aussetzt.

2. Verfahren nach Anspruch 1, dadurch gekennzeichnet, daß die Säuredämpfe ein Mischungsverhältnis von 1 Teil Persäure zu 1 - 4 Teilen des entsprechenden Säureanhydrids aufweisen.

## Claims

1. Process for the gentle sterilization of biological active substances, in particular organ tissues for therapeutic purposes, with regard to microorganisms and viruses, which are subjected to controlled chemical digestion using vapors and chemical reagents, in which the powder substrate and the digestive reagent, the boiling point of which is above standard temperature at atmospheric pressure, in particular concentrated sulfuric acid, acetic acid, formic acid or diethylamine, are exposed together but separated from each other, in a closed system to a vacuum that permits transformation of the digesting agent into the gaseous phase and, once the desired digestion of the substrate has been achieved, again removes the unconsumed reagent, whereby deep-frozen and powdered organic tissues in particular are assumed, and the gaseous chemical reagent of a predetermined quantity is first made to settle on the substrate by decreasing the vacuum and, once the desired reaction has taken place, the excess reagent is again removed by increasing the vacuum, characterized by the substrate being exposed in a vacuum to vapors of peracids or mixtures of peracids and the corresponding concentrated acids.

2. Process in accordance with claim 1 characterized by the acid vapors having a mixture ratio of 1 part peracid to 1 - 4 parts of the corresponding acid anhydride.

## Revendications

1. Procédé relatif à la stérilisation précautionneuse de principes actifs biologiques, en particulier de tissus d'organe destinés à l'usage thérapeutique, contre les microorganismes et les virus qui sont soumis à une désagrégation chimique contrôlée utilisant des vapeurs et des réactifs chimiques; dans le cadre du présent procédé les substrats pulvérulents et le réactif produisant la désagrégation dont le point d'ébullition se situe, sous pression atmosphérique, au-dessus de la température normale - il s'agit en particulier d'acide sulfurique concentré, d'acide acétique, d'acide formique ou de diéthylamine - sont soumis, dans un système fermé, mais séparé l'un de l'autre, à un vide permettant le transfert de la substance désagrégante dans la phase de vapeur; quand la désagrégation désirée du substrat est obtenue, le réactif non-utilisé est enlevé; ce procédé s'adresse avant tout aux tissus d'organe surgelés et pulvérisés; le réactif chimique sous forme de vapeur provenant d'une quantité prédéterminée est d'abord liquéfié sur le substrat par diminution du vide et après l'action désirée l'excédént du réactif est éliminé par augmentation du vide, c'est-à-dire le procédé est caractérisé par le fait que les substrats sont soumis, sous vide, à des vapeurs de peracides ou de mélanges composés de peracides et d'acides concentrés appropriés.

2. Procédé selon revendication 1 caractérisé par le fait que les vapeurs d'acide présentent les proportions de mélange suivantes: 1 part de peracide pour 1 à 4 parts de l'anhydride d'acide approprié.